# EUROPEAN PATENT APPLICATION

(11) **EP 2 239 553 A1**
(43) Date of publication of application: **13.10.2010**
(21) Application number: 09305310.6
(22) Date of filing: 09.04.2009
(51) Int. Cl.: G01N 1/02, G01N 33/24

(54) **A carbon compounds collecting system**

(71) Applicant: Total S.A., 92400 Courbevoie (FR)
(72) Inventor: Dessort, Daniel, 64018, Pau Cedex (FR); Duclerc, Dominique, 64018, Pau Cedex (FR); Le Van Loi, Robert, 64018, Pau Cedex (FR); Cassagne, Alain, 64018, Pau Cedex (FR)
(74) Representative: Cabinet Plasseraud

(57) **Abstract**

A collecting system for collecting at least part of the volatilisable or pyrolysable carbon compounds comprised in a core (10) taken from a well is disclosed. The system comprises:
• at least a heating device (32) comprising a laser source (36), the heating device (36) being configured so as to focus on the surface of the core (10) a laser beam issued from the laser source (36), the laser beam having at the surface of the core (10) a power density of at least 2 W/mm², so as to cause the volatilisation or the thermal decomposition of at least part of the hydrocarbons comprised in a core (10)
• at least a collecting device (34) configured to collect at least part of the substance released from the core (10) due to the volatilisation or the thermal decomposition of part of the hydrocarbons of the core (10) when the laser beam is focused at the surface of the core (10).

## Description

The invention relates to a collecting system for collecting part of the carbon compounds comprised in a core taken from a well and to a method for collecting part of the carbon compounds comprised in a core taken from a well.

In the field of geochemical research and petroleum exploration and production, an area of growing interest is analysis of geological samples to determine:
1/ the remaining petroleum generating potential of a rock sample.
2/ the presence of oil/heavy oil/bitumen or Tarmat in the petroleum reservoir.
   Work in this field has been directed to methods for collecting hydrocarbons from geological samples and analyzing the hydrocarbons, to apparatuses for laboratory and field analysis for geological samples, and to the development of theoretical and practical models for predicting petroleum exploration and production related characteristics for the raw data obtained.

Pyrolysis and Pyrolysis-gas chromatography (pyrolysis-GC) techniques have proven successful in providing useful information in determining kerogen characteristics, oil-source rocks correlations, petroleum generation kinetics, gas-oil ratios (GOR) and precursor product relationships with a view, among others applications, to determining catagenic petroleum yield and composition.

In performing analyses of the type described above, it is desirable to be able to make accurate, reproducible determinations of quantity of volatile and pyrolysable hydrocarbons present in the geological sample.

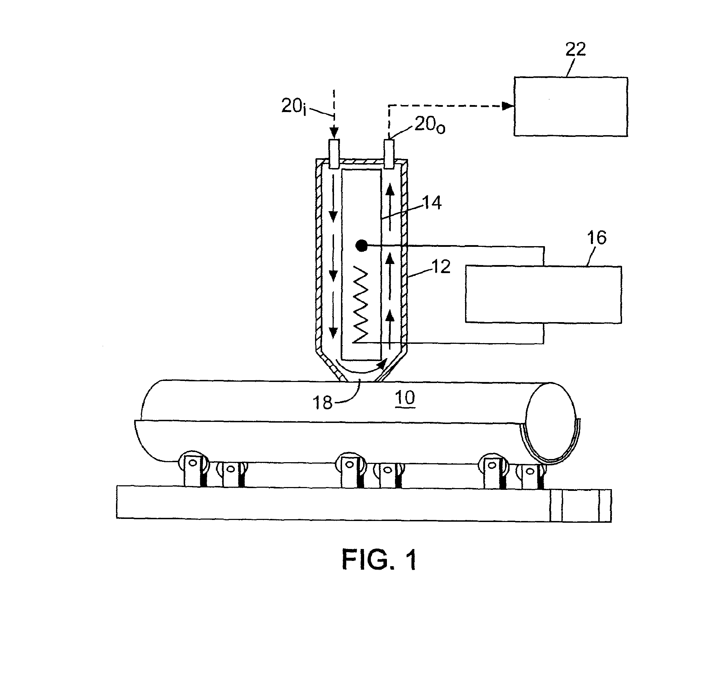

A known system for collecting hydrocarbon potential of a whole core 10 taken from a well is represented in fig 1.

Such system comprises a heat probe 12 heated by a heating cartridge 14 under control of a temperature controller 16 such as a thermocouple. The heat probe 12 is cylindrical and formed of heat conductive metal such as brass and has a core adjacent end or tip which is urged into contact with the core 10.

A cavity formed within the core-adjacent end of heat probe 12 forms a collection chamber 18. A carrier gas is introduced by line 20i into chamber 18, where it flows past the heated portion of core 10 absorbing evolved gases. The carrier gas is delivered by line 20o to a detector 22.

The hydrocarbon potential of the core is evaluated for example by contacting a portion of the core 10 with the heat probe 12, controlling the heat applied to the core 10 by the heat probe 12 in surface contact with the portion of surface of whole core 10 to a temperature effective for causing the pyrolysis and the release of hydrocarbons, causing at least a portion of released hydrocarbons to flow into the collection chamber 18 and collecting the released hydrocarbons.

Although such evaluating system may be efficient, it presents a certain number of drawbacks.

The system of fig 1 requires the use of a carrier gas to collect the released hydrocarbons; therefore such system requires a carrier gas source.

Furthermore, the system of fig 1 requires a heat regulating system to regulate the probe temperature.

The system of fig 1 requires a carrier gas source and a heat regulating system therefore making such system bulky.

A further drawback of the system of fig 1 is that the resolution of such system is limited by the size of the heating probe 12. Furthermore, heating the heat probe and the rock at different temperatures is time consuming since the heating probe and rock have heat inertia.

Accordingly, there remains a need for a high resolution and fast collecting system that would not present the drawbacks of known systems. Thus, a goal of the present invention is to improve the present situation.

The invention relates to a collecting system for collecting at least part of the volatilisable or pyrolysable carbon compounds comprised in a core taken from a well, the system comprises:
- at least a heating device comprising a laser source, the heating device being configured so as to focus on the surface of the core a laser beam issued from the laser source, the laser beam having at the surface of the core a power density of at least 2 W/mm², so as to cause the volatilisation or the thermal decomposition of at least part of the hydrocarbons comprised in a core,
- at least a collecting device configured to collect at least part of the substance released from the core due to the volatilisation or the thermal decomposition of part of the hydrocarbons of the core when the laser beam is focused at the surface of the core.

Advantageously, the use of a laser source as heating means for the heating device makes it possible to heat only a small area of the surface of the core therefore increasing the resolution of the collecting system.

Furthermore, using a laser source as heating source allows controlling efficiently and almost instantly the heating temperature. Therefore, the heat inertia of the heating device and the time required to change the heating temperature are reduced.

According to further embodiments which can be considered alone or in combination:
○ the heating device comprises at least a focusing device configured to focus the laser beam issued from the laser source,
○ the system further comprises at least one positioning device configured to position the heating means at a release distance of the surface of the core, the release distance being selected so has to have the power density of the laser beam at the surface of the core at least equal to 2 W/mm²,
○ the positioning device is configured to move the heating device along the surface of the core to collect at least part of the carbon compounds comprised in different parts of the core,
○ the laser beam issued from the laser source has a wavelength greater or equal to 0.78 µm and smaller or equal to 100 µm,
○ the laser source is a semiconductor laser source and the laser beam issued from the laser source is focused on the core surface via an optical fiber,
○ the heating device is configured so as to focus on the surface of the core a laser beam having a power density smaller or equal to 50 W/mm²,
○ the heating device is configured to locally heat an area of the surface of the core at a temperature of at least 300°C when the laser beam is focused at the surface of the core,
○ the heating device is configured so as to have the surface of impact of the laser beam on the core smaller or equal to 5 mm²,
○ the collecting device comprises at least one aspiration device configured to aspirate at least part of the substances released from the core due to the volatilization or the thermal decomposition of part of the hydrocarbons comprised in the core when the laser beam is focused at the surface of the core,
○ the collecting device comprises a tube, through which the laser beam may be focused on the surface of the core,
○ the system further comprises at least one quantification device configured to quantify the quantity of carbon compounds comprised in the collected substances.

According to another aspect, the invention relates also to a method of method for collecting at least part of the carbon compounds comprised in a core taken from a well using a collecting system according to the invention, the method comprises:
- a positioning step in which the heating device is positioned relative to the surface of a core so as to focus the laser beam at the surface of the core,
- a heating step in which the surface of the core is heated by the heating device of the collecting system so as to cause the thermal decomposition and the volatilisation of at least part of the hydrocarbons comprised in the core,
- a collecting step in which at least part of the substances released from the core due to the thermal decomposition or the volatilisation of part of the hydrocarbons comprised in the core are collected.

According to further embodiments, the method may comprise a moving step in which the heating device and the collecting device are moved along the surface of the core so as to collect at least a portion of the carbon compounds comprised in different parts of the core and/or further to the collecting step a quantification step in which the quantity of carbon compounds in the collected substances is quantified.

The invention also relates to a computer program product for a data acquisition and processing system, the computer program product comprises a set of instructions which, when loaded into the data processing system, causes the data processing system to perform at least one, for example all, of the steps, of the method according to the invention.

In addition, the present invention provides a computer-readable medium carrying one or more set of instructions of a computer program product of the invention.

Unless specifically stated otherwise, as apparent from the following discussions, it is appreciated that throughout the specification discussions the terms "computing", "processing", or the like, refer to the action and/or processes of a computer or computing system, or similar electronic computing system, that manipulate and/or transform data represented as physical, such as electronic, quantities within the computing system's registers and/or memories into other data similarly represented as physical quantities within the computing system's memories, registers or other such information storage, transmission or display systems.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer or Very high speed integrated circuit Hardware Description Language ("VHDL"), or Complex Instruction Set Computer ("CISC") architecture, or Reduced Instruction Set Computer ("RISC") architecture.

Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method. In addition, embodiments of the present invention are not described with reference to any particular programming language. It will be appreciated that a variety of programming languages may be used to implement the teachings of the inventions as described herein.

Non limiting embodiments of the invention will now be described with reference to the accompanying drawing wherein:
- Fig. 1 is a schematic representation of a collecting system according to the prior art;
- Fig. 2 is a schematic representation of a collecting system according to an embodiment of the invention.

Elements in the figures are illustrated for simplicity and clarity and have not necessarily been drawn to scale. For example, the dimensions of some of the elements in the figures may be exaggerated relative to other elements to help improve the understanding of the embodiments of the present invention.

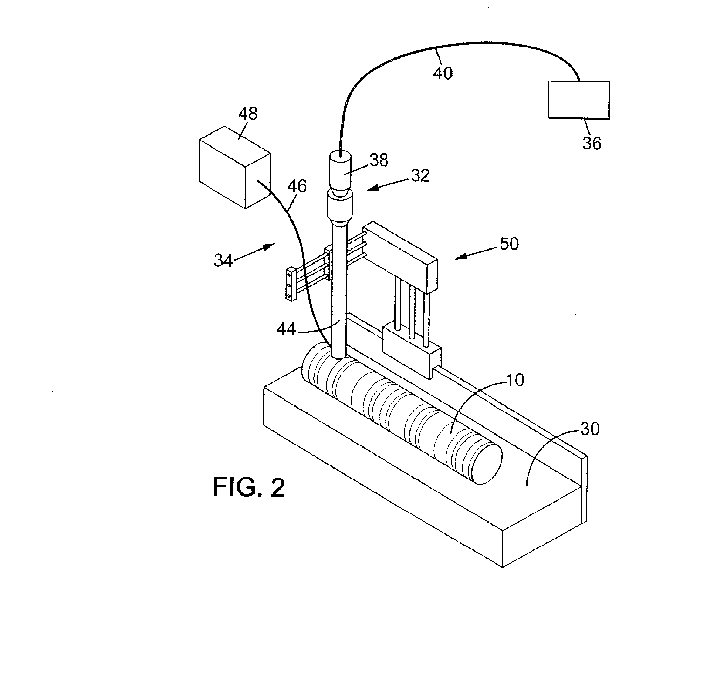

A collecting system according to an embodiment of the invention is represented on figure 2. The collecting system represented on figure 2 is adapted for collecting at least part of the carbon compounds comprised in a core 10 taken from a well. The collecting system comprises:
- a receiving device 30 configured to receive the core 10,
- a heating device 32 configured to cause the thermal decomposition or volatilisation of at least part of the hydrocarbons, comprised in the core 10,
- a collecting device 34 configured to collect at least part of the substances release from the core due to the thermal decomposition or volatilisation of part of the hydrocarbon of the core 10 when heated by the heating device 32.

According to an embodiment of the invention, the receiving device may comprise a core holder arranged to receive the core 10, for example a semi cylindrical core trough, as the one illustrated on fig 1.

According to an embodiment of the invention, the receiving device 30 may comprise a moving device arranged to move the core 10. The moving device may be arranged to translate the core 10 along a direction parallel to the longitudinal axis of the core 10.

According to an embodiment of the invention the moving device may be arranged so as to move the core 10 in increments using for example a motor having friction drive wheel. Advantageously, the core is moved in increments past the heating 32 and collecting 34 devices so as to collect part of the hydrocarbon comprised in different part in the core 10.

According to an embodiment of the invention, the heating device 32 comprise a laser source 36 and a focusing device 38 configured to focus the laser beam issued from the laser source 36. The heating device is configured so as to focus on the surface of the core 10 a laser beam issued from the laser source 36, the laser beam having at the surface of the core 10 a power density of at least 2 W/mm².

The inventors have observed that so as to efficiently cause the thermal decomposition of at least part of the hydrocarbon comprised in a core, a power density of at least 2 W/mm² was necessary.

Indeed, using a laser source having a power density smaller than 2 W/mm² may not allow the thermal decomposition of the hydrocarbon comprised in a core. Furthermore, the power density of the laser beam should be smaller or equal to 50 Watts/mm² so as to avoid the degradation of the core 10 as a whole.

The laser source 36 and the focusing device 38 are configured so as to cause the thermal decomposition of at least part of the hydrocarbon comprised in the core 10 when the laser beam is focused at the surface of the core 10.

According to an embodiment of the invention, the laser source is a semiconductor laser source.

According to an embodiment of the invention, the laser source 36 is linked to the focusing device by a waveguide 40, for example an optical fibre, so as to guide the laser beam to the focusing device 38.

Advantageously, such configuration of the laser source 36 allows using a given laser source 36 for different collecting systems according to the invention.

According to an embodiment of the invention, the laser source is chosen so as to have wavelength greater or equal to 0.78 µm and smaller or equal to 100 µm.

According to an embodiment of the invention, the focusing device 38 may comprise any focusing arrangement comprising optical components known from the person skilled in the art, for example, a system of optical lenses.

According to an embodiment of the invention, the focusing device 38 is arranged so as to have the focal length of said focusing device 38 smaller than 50 cm, for example about 30 cm.

According to an embodiment of the invention, the heating device 32 is arranged so as to locally heat an area of the surface of the core 10 at a temperature of at least 300°C when the laser beam is focused at the surface of the core 10.

Advantageously, heating the surface of the core 10 at a temperature of at least 300°C allows the thermal decomposition of at least part of the volatilisable or pyrolysable hydrocarbons comprised in a core 10.

In the sense of the invention the wording "volatilisable hydrocarbons" refers to saturated or aromatic hydrocarbons or organo-sulfur coumpounds having a molecular mass smaller or equal to 50 carbon atoms.

In the sense of the invention the wording "pyrolysable hydrocarbons or organic matter" refers to organic material having a molecular mass smaller or equal to 30 carbon atoms.

The accurate frontier between these domains is not well defined. For this reason, there is an overlap of the range of the molecular weight between them.

According to an embodiment of the invention, the heating device 32 is arranged so as to locally heat an area of the surface of the core at the temperature of at least 800°C when the laser beam is focused at the surface of the core 10.

Heating the surface of the core 10 at a temperature of about 800°C allows volatilisation, pyrolysis and expelling part of the hydrocarbons from the oil, bitumen or kerogen comprised in the core 10.

Advantageously, comparing the level of volatile hydrocarbons released from the core 10 at a temperature of about 300°C with the level of hydrocarbon released from the core 10 at a temperature of 800°C may be used to characterize the organic material and to determine the maturation level of the core 10.

According to an embodiment of the invention, the laser source 36 may comprise a power controller for controlling the power of said laser source 36 so as to incrementally change the power of the laser source so as to heat the surface of the core at different desired temperatures.

According to an embodiment of the invention, the laser source 36 and the focusing device 38 are configured so as to have the surface of impact of the laser beam on the surface of the core 10 smaller or equal to 5 mm², for example 2 mm², for example 1 mm².

Advantageously, having a surface of impact as small as possible, for example smaller or equal to 5 mm², allows increasing the resolution of the collecting system.

In the sense of the invention, the "surface of impact" of the laser beam on the surface of the core 10 corresponds to the surface of the intersection of the exterior surface of the core 10 with the laser beam issued from the focusing device 38.

According to an embodiment of the invention, the collecting device 34 may comprise an aspiration device configured to aspirate at least part of the substance released from the core due to the thermal decomposition of part of the hydrocarbon comprised in the core when the laser beam is focused at the surface of the core 10.

As illustrated on figure 2, the collecting device 34 may comprise a tube 44, for example a cylinder tube, through which the laser beam may be focused on the surface of the core 10.

According to different embodiments of the invention, the tube 44 may be hollow or may be field with a material transparent at the wavelength of the laser source 36.

The collecting device also comprises a flexible pipe adapted at the core-adjacent end of tube 44 and linked to an aspiration system 48.

The aspiration system 48 may also comprise a quantification device configured to quantify the quantity of carbon compounds comprised in the collected substances.

Any quantification device known from the person skilled in the art may be used, for example, mass spectrometer, UV detection, IR detection, chromatography, NMR, flame ionization detector, thermal conductivity detector.

Advantageously, a collecting system comprising a quantification device allows quantifying the amount of carbon compounds comprised in the substances released from the core when the laser beam is focused at the surface of the core 10.

The quantification device may be calibrated using any known calibration process.

The collecting system according to the invention may also comprise a positioning device 50 configured to position the heating device 32 relative to the surface of the core 10, for example, positioning the focusing device 38 at the focusing length from the surface of the core 10.

According to the embodiment illustrated on fig 2, the collecting device 34 and the focusing device 38 are linked together via the tube 44. The positioning device 50 is arranged to control the position of the tube 44 relative to the surface of the core 10.

The positioning device may be arranged to as to incrementally move the heating 32 and collecting 34 devices along the surface of the core 10. Moving the heating 32 and collecting 34 devices relative to the core 10 allows collecting part of the hydrocarbon comprised in different parts in the core 10.

The heating 32 and collecting 36 devices being lighter than the core 10, it is easier to move the heating and collecting devices relative to the surface of the core than to move the core 10 relative to the heating 32 and collecting 36 devices.

Advantageously, the collecting system according to the invention is compact, light and easily moveable. Therefore, the collecting system according to the invention may be used in the field.

According to an embodiment of the invention, the collecting system may further comprise at least one observation device (not represented) configured to observe the impact mark left by the impact of the laser beam on the surface of the core 10. The observation device may comprise a CCD camera placed vertically above the core so as to take a picture of the impact mark. The observation device may be linked to the heating 32 and collecting 34 devices and moved along the surface of the core 10 together with the heating and collecting devices.

The inventors have observed that data related to the impact mark, for example the size and/or the colour of the impact mark may provide information concerning the core 10, for example information concerning the porosity and permeability of the core 10.

The invention further relates to a method for collecting at least part of the carbon compounds comprised in a core 10 taken from a well using a collecting system according to the invention.

According to an embodiment of the invention the collecting method may comprises:
- a positioning step in which the heating device 32 is positioned relative to the surface of a core 10 so as to focus the laser beam at the surface of the core 10;
- a heating step in which the surface of the core 10 is heated by the heating device 32 of the collecting system so as to cause the thermal decomposition of at least part of the hydrocarbons comprised in the core 10, for example by heating the surface of the core 10 at a temperature of 300°C;
- a collecting step in which at least part of the substances released from the core due to the volatilisation or the thermal decomposition of part of the hydrocarbons comprised in the core 10 are collected, for example by aspiration;
- a quantification step in which the quantity of carbon compounds in the collected substances is quantified, for example using a UV detector; and
- a moving step in which the heating device 32 and collecting device 34 are moved along the longitudinal axe of the core 10 so as to collect at least a portion of the carbon compounds comprised in different parts of the core 10.

Prior to the collecting method of the invention, the quantification device may be calibrated according to any calibration process known from the person skilled in the art.

The quantity of carbon compounds collected at the different parts and temperature of the core may be recorded, for example in a data file. Such data file may be used to determine a log of the quantity of volatilisable hydrocarbons in the different parts of the core.

According to an embodiment of the invention, the method may further comprise before the moving step a second heating step. According to such embodiment of the invention the method further comprise prior to the moving step:
- a second heating step in which the surface of the core 10 is heated by the heating device 32 of the collecting system so as to cause the thermal decomposition of at least part of the hydrocarbons comprised in the core 10, the heating temperature being greater than the one of the first heating step, for example the surface of the core 10 is heated at a temperature of 800°C;
- a collecting step in which at least part of the substances released from the core due to the thermal decomposition of part of the hydrocarbons comprised in the core 10 are collected, for example by aspiration;
- a quantification step in which the quantity of carbon compounds in the collected substances is quantified, for example using a UV detector; and

As indicated here above, comparing the level of volatile hydrocarbons released from the core 10 at a temperature of about 300°C with the level of hydrocarbon released at a temperature of about 800°C may be used to characterize and quantify the carbonaceous material and to determine the maturation level of the core at a given point.

According to an embodiment of the invention, the method further comprises after the moving step a observation step in which the impact mark is observed, for example a image of the impact mark is recorded.

Information concerning the impact mark, for example the size and/or colour, may be recorded, for example in a data file. Such data file may be used to determine a log of the porosity and porosity or physical properties of the fluid (density, viscosity) of the different parts of the core.

The invention has been described above with the aid of embodiments without limitation of the general inventive concept.

## Claims

1. A collecting system for collecting at least part of the volatilisable or pyrolysable carbon compounds comprised in a core (10) taken from a well, the system comprises:
• at least a heating device (32) comprising a laser source (36), the heating device (36) being configured so as to focus on the surface of the core (10) a laser beam issued from the laser source (36), the laser beam having at the surface of the core (10) a power density of at least 2 W/mm², so as to cause the volatilisation or the thermal decomposition of at least part of the hydrocarbons comprised in a core (10)
• at least a collecting device (34) configured to collect at least part of the substance released from the core (10) due to the volatilisation or the thermal decomposition of part of the hydrocarbons of the core (10) when the laser beam is focused at the surface of the core (10).

2. The system according to claim 1, wherein the heating device (32) comprises at least a focusing device (38) configured to focus the laser beam issued from the laser source (36).

3. The system according to any of claims 1 or 2, wherein the system further comprises at least one positioning device (50) configured to position the heating means (32) at a release distance of the surface of the core, the release distance being selected so has to have the power density of the laser beam at the surface of the core (10) at least equal to 2 W/mm².

4. The system according to claim 3, wherein the positioning device (50) is configured to move the heating device (32) along the surface of the core to collect at least part of the carbon compounds comprised in different parts of the core (10).

5. The system according to any of the preceding claims, wherein the laser beam issued from the laser source (36) has a wavelength greater or equal to 0.78 µm and smaller or equal to 100 µm.

6. The system according to any of the preceding claims, wherein the laser source (36) is a semiconductor laser source and the laser beam issued from the laser source is focused on the core surface via an optical fiber (40).

7. The system according to any of the preceding claims, wherein the heating device (32) is configured to locally heat an area of the surface of the core (10) at a temperature of at least 300°C when the laser beam is focused at the surface of the core (10).

8. The system according to any of the preceding claims, wherein the heating device (32) is configured so as to have the surface of impact of the laser beam on the core smaller or equal to 5 mm².

9. The system according to any of the preceding claims, wherein the collecting device (34) comprises at least one aspiration device configured to aspirate at least part of the substances released from the core due to the volatilization or the thermal decomposition of part of the hydrocarbons comprised in the core (10) when the laser beam is focused at the surface of the core (10).

10. The system according to any of the preceding claims, wherein the system further comprises at least one quantification device configured to quantify the quantity of carbon compounds comprised in the collected substances.

11. Method for collecting at least part of the carbon compounds comprised in a core taken from a well using a collecting system according to any of claims 1 to 10, the method comprises:
• a positioning step in which the heating device (32) is positioned relative to the surface of a core (10) so as to focus the laser beam at the surface of the core (10),
• a heating step in which the surface of the core (10) is heated by the heating device (32) of the collecting system so as to cause the volatilisation or the thermal decomposition of at least part of the hydrocarbons comprised in the core (10),
• a collecting step in which at least part of the substances released from the core (10) due to the volatilisation or the thermal decomposition of part of the hydrocarbons comprised in the core (10) are collected.

12. The method according to claim 11, wherein the method further comprises a moving step in which the heating device (32) and the collecting device (34) are moved along the surface of the core so as to collect at least a portion of the carbon compounds comprised in different parts of the core (10).

13. The method according to claim 11 to 12, wherein the method comprises further to the collecting step a quantification step in which the quantity of carbon compounds in the collected substances is quantified.

14. A computer program product comprising one or more stored sequence of instruction that is accessible to a processor and which, when executed by the processor, causes the processor to carry out the steps of any of claims 11 to 13.

15. A computer readable medium carrying one or more sequences of instructions of the computer program product of claim 14.
